# EUROPÄISCHE PATENTSCHRIFT

(11) **EP 3 840 734 B1**
(45) Veröffentlichungstag und Bekanntmachung des Hinweises auf die Patenterteilung: **20.07.2022**
(21) Anmeldenummer: 19765417.1
(22) Anmeldetag: 22.08.2019
(51) Int. Cl.: A61K 9/28, A61K 31/58, A61K 9/50, A61K 9/00

(54) **PELLETS MIT MEHRSCHICHTIGER STRUKTUR ZUR VERZÖGERTEN FREISETZUNG DES WIRKSTOFFS IM DISTALEN KOLON**
PELLETS WITH MULTILAYER STRUCTURE FOR DELAYED RELEASE OF THE DRUG IN THE DISTAL COLON
GRANULÉS À STRUCTURE MULTICOUCHE PERMETTANT LA LIBÉRATION RETARDÉE DE LA SUBSTANCE ACTIVE DANS LE COLON DISTAL

(30) Priorität: 24.08.2018 EP 18190638
(43) Veröffentlichungstag der Anmeldung: 30.06.2021
(73) Patentinhaber: Dr. Falk Pharma GmbH, 79108 Freiburg (DE)
(72) Erfinder: WILHELM, Rudolph, 76476 Bischweier (DE); PRÖLS, Markus, 79100 Freiburg/Breisgau (DE); GREINWALD, Roland, 79341 Kenzingen (DE); NACAK, Tanju, 79288 Gottenheim (DE); BÖGERSHAUSEN, Ansgar, 79102 Freiburg (DE)
(74) Vertreter: Lederer & Keller Patentanwälte Partnerschaft mbB
(86) Internationale Anmeldenummer: PCT/EP2019/072429
(87) Internationale Veröffentlichungsnummer: WO 2020/039017

(56) Entgegenhaltungen:
- EP-A1- 1 607 087
- EP-A1- 2 143 424
- WO-A1-2004/039357
- WO-A1-2017/042835
- WO-A2-03/080032
- DE-A1- 4 332 394
- DE-A1- 4 340 057
- US-A- 5 643 602

## Beschreibung

Für die Behandlung von entzündlichen Prozessen und Veränderungen des distalen Kolons, wie zum Beispiel der Colitis ulcerosa, ist eine Arzneiform erforderlich, die nach oraler Anwendung die lokale Verfügbarkeit des Wirkstoffes Budesonid in genügend hoher Konzentration am Entzündungsort ermöglicht. Dieses auch als Kolon-Targeting bezeichnete Konzept ist mit der oralen Applikation einer herkömmlichen pharmazeutischen Formulierung nicht realisierbar, da ein hohes Risiko besteht, dass der Wirkstoff nicht in effektiven Konzentrationen am Wirkort, den krankhaft veränderten Bereichen des Kolons, zur Verfügung gestellt werden kann. Das Ziel des Kolon-Targeting soll daher bevorzugt dadurch erreicht werden, dass der Wirkstoff aus der Formulierung mit einer modifizierten Freisetzungskinetik möglichst vollständig im Kolon freigesetzt wird und dort wirken kann. Die vorliegende Erfindung ist speziell geeignet, eine Arzneiform zur Verfügung zu stellen, die einfach und verlässlich anzuwenden ist und diese Anforderungen erfüllt.

Im Stand der Technik sind verschiedenste Formulierungen zur Behandlung von entzündlichen Darmerkrankungen beschrieben worden. Es gibt verschiedene Ausprägungen von entzündlichen Darmerkrankungen, wobei sich Morbus Crohn an den verschiedenen Bereichen des Darms manifestieren kann. Es gibt Erscheinungsformen, bei denen ein Schwerpunkt des entzündlichen Bereichs im Duodenum liegt. Bei anderen Formen liegt es im Jejunum oder Ileum oder auch im Dickdarm. Bei manchen Patienten erstrecken sich die befallenen Bereiche über weite Teile des Darms. Colitis ulcerosa manifestiert sich dagegen fast ausschließlich im Dickdarm, wobei auch der Dickdarm in verschiedene Bereiche unterteilt wird. In den meisten Fällen ist das Rektum betroffen, von wo aus sich die Erkrankung in das Sigma und Kolon fortsetzt.

Für die pharmazeutische Technologie ist es eine Herausforderung, den Wirkstoff möglichst genau an derjenigen Stelle des Darmes zur Verfügung zu stellen, an der der Wirkstoff gebraucht wird. Diese Aufgabe, den Wirkstoff ganz überwiegend im Kolon freizusetzen, wird von den im Stand der Technik bekannten Formulierungen nicht zufriedenstellend gelöst.

In der WO 91/07172 werden oral verabreichbare Zusammensetzungen für die Behandlung von entzündlichen Darmerkrankungen beschrieben, wobei einer der Wirkstoffe Budesonid ist. Aufgebaut sind die dort beschriebenen Pellets so, dass eine Schicht des Wirkstoffes auf Starter-Kerne aufgebracht wird, wobei diese von zwei verschiedenen Schichten umhüllt wird, die auf die Trägerpellets mit der Wirkstoffschicht aufgesprüht werden. In der WO 95/08323 werden Budesonid-Pellets mit einem kontrollierten Freisetzungsmuster offenbart. Diese Pellets beinhalten von innen nach außen Neutralpellets, eine Wirkstoffschicht, eine innere Lackschicht aus darmsaftlöslichen Lacken und eine äußere Lackschicht aus magensaftunlöslichen, darmsaftlöslichen Lacken. Die dadurch erzielbare Freisetzung des Wirkstoffes nach der Magenpassage ist für die beschriebene Indikation (Colitis ulcerosa) nicht zufriedenstellend nutzbar. Die gewünschte verzögerte, kontinuierliche Freisetzung des Wirkstoffes im Kolon wird nicht erreicht. Die im Stand der Technik beschriebenen Pellets enthaltenden Hartkapseln weisen eine frühe, sofortige und rasche Freisetzung auf, die dazu führt, dass nur wenig Wirkstoff das Kolon überhaupt erreicht.

Pharmazeutische Formulierungen mit mehrfachen Beschichtungen werden auch in WO 03/045356 oder WO 2017/216088 offenbart, wobei auch mucoadhäsive Materialien vorgesehen sind. Weitere Formulierungen zur kontrollierten Freisetzung sind in der WO 2009/138716 oder in der WO 00/76478 offenbart, wobei die Wirkstoffe teilweise in dreidimensionale Matrices eingearbeitet sind, wodurch eine verzögerte Freisetzung bewirkt wird. Bekannt sind auch komplexe Arzneiformulierungen, die den Wirkstoff aus einer Matrix kontinuierlich freisetzen (Cortiment-MMX Retardtabletten,). Die WO 02/17887 offenbart Arzneimittel zur Behandlung von Darmerkrankungen, wobei der Wirkstoff vorwiegend in den distalen Darmabschnitten freigesetzt wird. Es handelt sich hierbei um Pellets, Granulate oder Minitabletten, welche vorzugsweise 5-Aminosalicylsäure enthalten und einzeln überzogen sind mit einem magensaftresistenten Lack und mit einem zweiten Lack.

In der EP 2 143 424 werden pharmazeutische Formen für die Kolonspezifische Verabreichung offenbart. Die Zusammensetzung umfasst einen Kern, der von einer Schicht mit dem Wirkstoff umhüllt ist. Darauf aufgebracht ist eine Zwischenschicht aus kationischem Polymer, das bei einem pH-Wert von nicht mehr als 6,6 quellbar ist und eine äußere Schicht die ein anionisches Polymer ist, das bei einem pH von nicht weniger als 7,0 löslich ist. Die Freisetzung des Wirkstoffes erfolgt nach einer Verzögerung von etwa 300 Minuten relativ schnell, schlagartig und vollständig.

Die DE 43 32 394 beschreibt Budesonid-Pellets mit kontrolliertem Freigabeprofil, wobei die Neutralpellets eine Wirkstoffschicht aus Budesonid und Hilfsstoffen aufweisen und zwei verschiedenen Lackschichten umfassen.

WO 03/080032 beschreibt pharmazeutische Formulierungen, die im Wesentlichen einen Kern mit dem Wirkstoff Budesonid, eine mittlere Schicht mit einem darmsaftlöslichen polymeren Überzugsmittel und eine äußere magensaftresistente Umfüllung umfasst.

EP 1 607 087 offenbart orale Formulierungen für die Verabreichung ans Kolon.

Gross et al., Journal of Crohn's and Colitis (2011) 5, 129-138 berichten über eine Studie, bei der sich herausgestellt hat, dass die Behandlung mit 3 g Mesalazin-Granulen (Salofalk^{®}) bessere Ergebnisse liefert als eine Behandlung mit 9 mg oral verabreichtem Budesonid, weil bei dem aus den Stand der Technik bekannten Budesonid-Präparaten die Freisetzung im Kolon nicht zuverlässig gewährleistet werden konnte.

Den aus dem Stand der Technik bekannten pharmazeutischen Formulierungen ist gemeinsam, dass die gewünschte Freisetzung des Wirkstoffes Budesonid nicht so gewährleistet wird, dass der Wirkstoff nahezu vollständig an den entzündeten Bereichen im gesamten Dickdarm, und insbesondere auch im Enddarm, zur Verfügung steht.

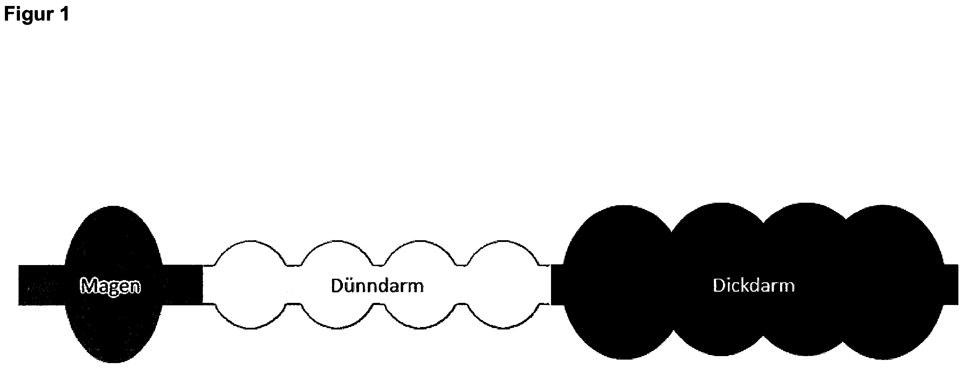

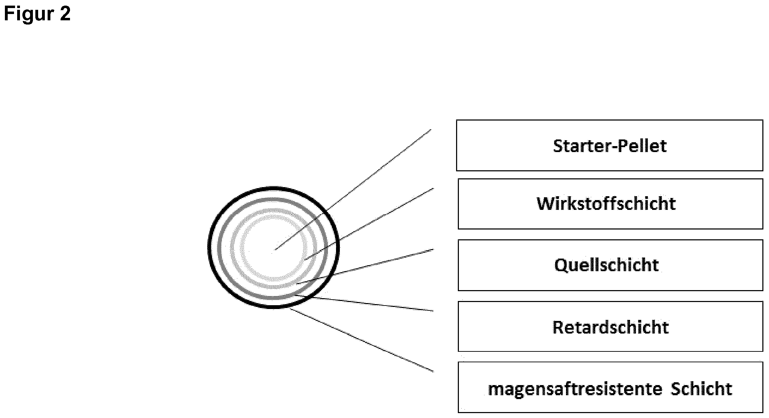

Gegenstand der vorliegenden Erfindung sind Pellets zur verzögerten und kontinuierlichen Freisetzung des Wirkstoffs im gesamten Kolon (Figur 1) mit einer mehrschichtigen Umhüllung, wobei das Pellet folgende Komponenten aufweist (Figur 2):
a) ein Starter-Pellet, das nur aus einem inerten Material besteht und keinen pharmazeutisch aktiven Wirkstoff im Inneren des Starter-Pellets aufweist,
b) eine Wirkstoffschicht, die direkt auf das Starter-Pellet aufgebracht ist und außer dem Wirkstoff lediglich in der Pharmazeutik gängige Hilfsstoffe enthält,
c) eine Quellschicht, die direkt auf die Wirkstoffschicht aufgebracht ist und Quellmaterialien enthält, die bei Kontakt mit Darmflüssigkeit aufquellen,
d) eine Retardschicht, die sich auch in Darmflüssigkeit bei einem pH-Wert >6,5 nicht auflöst, aber für Flüssigkeiten permeabel wird und direkt auf der Quellschicht c) aufgebracht ist,
e) eine äußerste Beschichtung, die sich bei einem pH-Wert <5,5 nicht auflöst, sich aber bei einem pH-Wert, der größer ist als 6,0, gut auflöst.

In bevorzugter Ausführungsform befinden sich zwischen den einzelnen Schichten wie oben aufgeführt keine weiteren Zwischenschichten. Dies bedeutet, dass die Wirkstoffschicht (b) direkt auf das Starter-Pellet (a) aufgebracht ist und, dass direkt auf der Wirkstoffschicht eine Quellschicht (c) aufgebracht ist. Auf die Quellschicht wird direkt eine Beschichtung (d) aufgebracht, die eine Retardschicht ist und direkt darauf wird die äußerste Beschichtung (e) aufgebracht, die magensaftresistent ist.

Die vorliegende Erfindung betrifft eine pharmazeutische Formulierung, die als wirksamen Bestandteil Budesonid oder ein pharmazeutisch verträgliches Salz davon umfasst und bevorzugt zur Behandlung von entzündlichen Erkrankungen des Kolons angewendet wird.

Die eingesetzten Starter-Pellets (a) haben einen mittleren Durchmesser von 0,2 bis 2,0 mm, wobei wenigstens 90 % der Teilchen innerhalb des angegebenen Größenbereichs liegen. Außerdem weisen die Starter-Pellets eine Kugelform mit gleichmäßiger Oberflächenbeschaffenheit auf.

Pharmazeutische Pellets sind Pulverpartikel von annährend kugeliger Form mit einem mittleren Durchmesser von 0,2 bis 2 mm, gleichförmiger Oberfläche und enger Teilchengrößenverteilung. Die Oberfläche ist glatt und wenig porös.

Wichtig ist, dass die als Starter-Pellet verwendeten Kügelchen bestimmte qualitative Anforderungen erfüllen. Als Pellet-Material kommen Zuckerpellets in Frage, die aus Saccharose und Maisstärke bestehen, wobei der Saccharose-Gehalt nicht höher als 90 Gew.% sein sollte. Die Pellets weisen bevorzugt einen engen Bereich der Teilchengrößenverteilung auf, weil dies eine der Voraussetzungen für eine homogene Verteilung der fertigen Pellets ist. Die Teilchen weisen einen Durchmesser zwischen 0,2 und 2,0 mm, bevorzugt 0,7 bis 1,4 mm auf, wobei ein Bereich zwischen 0,85 und 1,0 mm besonders bevorzugt ist. Bei der Herstellung der Pellets ist darauf zu achten, dass die Größenverteilung sich in einem relativ engen Bereich bewegt, so dass beispielsweise wenigstens 90 %, bevorzugt wenigstens 95 % der Teilchen sich in dem angegebenen Bereich befinden. Die Methoden zur Bestimmung der Partikelgröße und der Bestimmung der Verteilung der Teilchengröße sind im europäischen Arzneibuch beschrieben. Wir verweisen diesbezüglich auf das dortige Kapitel 2.9.38.

Ein anderer wesentlicher Aspekt betrifft die Oberflächenbeschaffenheit. Die Starter-Pellets sollen möglichst einer idealen Kugel entsprechen, wobei die Oberfläche möglichst glatt und ohne Vertiefungen oder herausstehende Vorsprünge sein soll. Die Qualität der Pellets kann beispielsweise mit Hilfe eines Stereo-Mikroskops und einer daran gekoppelten Digitalkamera überwacht werden. Die Bilder können mit Hilfe einer geeigneten Software ausgewertet werden.

Erfindungsgemäß wird als Wirkstoff das topisch wirksame Corticosteroid Budesonid eingesetzt. Budesonid ist kaum wasserlöslich. Daher wird in einer bevorzugten Ausführungsform mikronisiertes Budesonid verwendet.

Für die Anwendung besonders geeignet ist die Verwendung von mikronisiertem Budesonid (Spezifikation der Partikelgrößenverteilung: 100% < 10 µm und ≥ 95% < 5 µm; Bestimmungsmethode: Laserbeugung). Die physikalisch-chemischen Eigenschaften von Budesonid sind bekannt (Literatur: z. B. Merck Index, Arzneibuchkommentar):
- Weißes bis fast weißes kristallines Pulver
- Die Löslichkeit in wässrigen Systemen ist praktisch pH-unabhängig und beträgt 0,014 mg/ml.
- Spezifische Drehung [α]^{D}₂₀ von Epimer A beträgt +98,9 (0,28%; in Dichlormethan)
- pKa-Wert: 12,85 ± 0.10
- Schmelzpunkt: 221°C - 232°C.

Bei der Auswahl der erfindungsgemäßen Darreichungsform wird davon ausgegangen, dass die kontinuierliche Freisetzung aus der intakten Arzneiform erfolgt und, dass die Passage- und Transitzeiten im Magen-Darm-Trakt nicht ungünstig beeinflusst werden (z. B. durch längere Verweilzeiten im Magen nach Nahrungsaufnahme). Die Dosis des arzneilich wirksamen Bestandteils kann über eine weitgehend homogene aber große äußere Oberfläche verteilt und flexibel eingestellt werden. Diese Eigenschaften werden mit der erfindungsgemäßen multipartikulären Darreichungsform, nämlich den Pellets, erreicht. Die Abfüllung einer definierten Pelletmenge in Hartkapseln oder Portionspackungen, wie z. B. Stickpacks, erlauben dabei die Einstellung, Gabe und Anwendung einer definierten Dosis von Budesonid. Die in der besonders bevorzugten Ausführungsform beschriebenen Eigenschaften der Arzneiformulierung erlauben die variable Einzeldosisverabreichung von Budesonid in einer Menge von 3 bis 9 mg.

Die erfindungsgemäße Anwendung der Budesonid enthaltenden Arzneiformulierung reduziert deutlich das Risiko einer unerwünschten Aufnahme von Budesonid in den systemischen Kreislauf. Die multipartikuläre Darreichungsform hat gegenüber von im Stand der Technik beschriebenen "single-unit"-Formulierungen, den Vorteil, den Wirkstoff reproduzierbar und robust, das heißt weniger variabel, über einen weiten Bereich des entzündeten Kolon freizusetzen und ist daher für die Behandlung der von einer Entzündung betroffenen Darmabschnitte besonders geeignet. Die zu verabreichende Budesonid-Dosis kann so über eine große Oberfläche verteilt verabreicht werden, wobei sich diese Oberfläche im Bereich des Kolons befindet.

Eine vorzeitige Freisetzung des arzneilich wirksamen Bestandteils aus der erfindungsgemäßen Darreichungsform im Magen wird durch Magensaftresistenz der äußersten Beschichtung (e) vermieden. Aufgrund dieser magensaftresistenten äußersten Beschichtung (e) wird die äußerste Umhüllung des Pellets im Magen nicht aufgelöst. Im Magen herrscht ein pH-Wert von etwa 1 bis maximal etwa pH 5. Solange sich die Pellets im Magenbereich befinden, löst sich die äußerste Umhüllungsschicht nicht. Erst nach Übertritt in den Dünndarm erhöht sich der pH-Wert und die äußerste Beschichtung (e) löst sich auf. Im Dünndarm und in Abhängigkeit von der Zeit dringt intestinale Flüssigkeit durch die zweitäußerste Umhüllungsschicht (d) in das Innere der Pellets. Die Retardschicht (d) löst sich bei Kontakt mit der Darmflüssigkeit nicht auf. Auf Grund ihrer Permeabilität kommt die Flüssigkeit aber in Kontakt mit der darunterliegenden Quellschicht (c). Durch den Flüssigkeitskontakt beginnt die Quellschicht (c) zu quellen und ihr Volumen zu vergrößern. Dabei steigt der Druck im Inneren der Pellets und auf die Retardschicht. Dieser Druck führt dazu, dass sich Öffnungen bzw. Fehlstellen in der Retardschicht bilden, durch die gelöstes Budesonid aus der innersten Wirkstoffschicht (a) nach außen diffundieren kann. Bei der weiteren Passage der Pellets durch den unteren Verdauungstrakt setzt sich dieser Prozess kontinuierlich fort, so dass letztendlich eine vollständige Budesonid-Freisetzung aus den Pellets durch die so geöffnete Retardschicht ermöglicht wird.

Der Start der Freisetzung erfolgt erst bei einem pH-Wert von > 6 mit einer kurzen Verzögerungszeit. Anschließend wird der Wirkstoff kontinuierlich und pH-unabhängig über einen Zeitraum von ca. 9 Stunden freigesetzt, so dass am Ende eine nahezu vollständige Freisetzung von Budesonid aus den Pellets erfolgt ist, was einer Menge von mehr als 85% der deklarierten Wirkstoffmenge entspricht. Wesentlich hierbei ist, dass die Freisetzung des Wirkstoffes nicht schlagartig oder in einem kurzen Zeitintervall von 1 bis 2 Stunden sondern mehr oder weniger gleichmäßig in einem Zeitraum von etwa 8-10 Stunden, bevorzugt von 9 Stunden erfolgt.

Die Freisetzung des Wirkstoffes Budesonid aus Pellets gemäß der vorliegenden Erfindung wird in den Beispielen durch folgende *in vitro* Freisetzung bestimmt:

| | | |
|---|---|---|
| • Prüfapparatur: | Blattrührerapparatur (Apparatur 2 des europäischen Arzneibuchs) | |
| • Rührgeschwindigkeit: | 75 Umdrehungen/Minute | |
| • Prüfmedien: | | |
| (a) Künstlicher Magensaft: | (Simulated gastric fluid, SGF) | |
| | Medium: | 0,1 N HCl, pH 1, 0,1% Polysorbat 80 |
| | Volumen: | 900 ml |
| | Prüfzeit: | 2 Stunden |
| | Kriterium: | Magensaftresistenz (keine Freisetzung) |
| (b) Künstlicher Darmsaft: | (Simulated intestinal fluid, SIF) | |
| | Medium: | Phosphatpuffer mit 0,1% Polysorbat 80 und einer Osmolalität von ungefähr 270 mOsmol/kg sowie einem pH-Wert von 6,5. |
| | Volumen: | 900 ml |
| | Prüfzeit: | 16 Stunden |
| | Kriterium: | Vollständige Freisetzung |

Bei den einzelnen Messversuchen wurden die Pellets zunächst für zwei Stunden in künstlichem Magensaft belassen. Da hier ein pH-Wert von 1,2 eingestellt wurde, zeigt eine mangelnde Freisetzung des Wirkstoffs Budesonid die Intaktheit der Pellets. Nach zwei Stunden wurden die Pellets entnommen und in künstlichen Darmsaft mit einem pH-Wert von 6,5 überführt. Gemessen wurde die Freisetzung des Budesonids in das Medium. Die erfindungsgemäßen Pellets zeichnen sich dadurch aus, dass während der ersten zwei Stunden (in künstlichem Magensaft) praktisch kein Budesonid freigesetzt wurde. Nach zwei Stunden (in künstlichem Darmsaft) begann eine kontinuierliche Freisetzung von Budesonid, die ein "Kolon Targeting" ermöglichte.

Bei Überprüfung von Formulierungen, die aus dem Stand der Technik bekannt sind, wurde eine Freisetzung von Budesonid teilweise schon im Magensaft beobachtet. Andererseits wurde auch im Darmsaft eine schnelle Freisetzung gerade in den ersten Stunden beobachtet. Ein derartiges Freisetzungsprofil ist für den angestrebten Verwendungszweck nicht brauchbar, weil das Budesonid vor allem im Dünndarm und eventuell in den anfänglichen Dickdarmbereichen erfolgt, nicht aber - wie gewünscht - hauptsächlich im Kolon.

Für die Herstellung von pharmazeutischen Pellets sind vor allem zwei unterschiedliche Herstellungsverfahren beschrieben: Beschichten von Starterkernen (Mehrschichtpellets) sowie Feucht- und Schmelzextrusion. Mehrschichtpellets (erfindungsgemäß) sind mehrfach beschichtete Starterpellets, wobei die Starterkerne zum Beispiel wirkstoffbeladene Zucker-Stärke-Pellets (so genannte Nonpareille) sind. Der Wirkstoff wird dabei zunächst als eine eigene Schicht auf die Oberfläche der Nonpareilles aufgesprüht bevor weitere funktionelle Schichten aufgetragen werden, die die Freisetzung des Wirkstoffs modifizieren. Das Aufbringen bzw. Aufsprühen der einzelnen Schichten erfolgt dabei kontinuierlich in der Wirbelschicht, so dass die Pellets ihre spezifische Funktionalität erhalten.

Erfindungsgemäß werden für das Kolon-Targeting von Budesonid Mehrschichtpellets verwendet. Der Aufbau der entwickelten Mehrschichtpellets erfolgt dabei nach folgendem komplexen Schema:

| Zucker-Stärke-Pellets als inerte Starterpellets | | |
|---|---|---|
| Größe/Durchmesser: 0.85 - 1.00 mm | | |
| | ↓ | |
| Schicht 1: | Beladung mit Budesonid = | |
| | Wirkstoffpellets | |
| | ↓ | |
| Schicht 2: | Beladung mit einer Quellschicht | |
| | ↓ | |
| Schicht 3: | Beladung mit einer Retardschicht | |
| | ↓ | |
| Schicht 4: | Beladung mit einer magensaftresistenten | |
| | Schicht = Magensaftresistente Pellets | |
| | ↓ | |
| Abfüllung in Hartgelatinekapseln oder Stickpacks | | |

Überraschenderweise konnte gezeigt werden, dass nur der komplexe und systematische Aufbau der erfindungsgemäßen Pellets über Starterpellets, Wirkstoffpellets, Quellschichtpellets, Retardpellets und magensaftresistenten Pellets geeignet ist, den Transport zum Kolon sowie die anschließende kontinuierliche Freisetzung von Budesonid im Kolon sicherzustellen.

Neben diesem strengen strukturellen Aufbau der Mehrschichtpellets ist die Auswahl von geeigneten Hilfsstoffen für die einzelnen Schichten ein weiteres entscheidendes Merkmal der erfindungsgemäßen Anmeldung. Erst durch das Zusammenspiel von Struktur und Zusammensetzung ist es möglich, die Vorteile der multipartikulären Darreichungsform für den Wirkstoff Budesonid unter Berücksichtigung des erforderlichen Kolon-Targetings optimal auszunutzen.

Die einzelnen Schichten der bevorzugten erfindungsgemäßen Pellets beginnend mit den Starterkernen haben folgende qualitative Zusammensetzung:
- Wirkstoffpellets
   Der Wirkstoff Budesonid wird in einer organisch-wässrigen Suspension bestehend aus Wasser und Isopropylalkohol (Masseverhältnis von 80% w/w und 20% w/w) sowie den weiteren Bestandteilen Laktose-Monohydrat (Funktion: Füllmittel), Polyvinylpyrrolidon (PVP) des Typs Kollidon^{®} K25 (Funktion: Bindemittel), Polyoxyethylen(20)-sorbitan-monooleat (Polysorbat^{®} 80; Tween 80) (Funktion: Benetzungsmittel) und Talkum (Funktion: Trennmittel) auf die Starterpellets (Zuckerstärkepellets) aufgetragen. Der Feststoffanteil der Suspension beträgt ca. 27% w/w. Nach Beladen und Trocknen der Pellets haftet Budesonid an den Starterpellets.
- Quellschichtpellets:
   Die Wirkstoffpellets werden anschließend mit einer alkoholischen Suspension von homopolymerer, quervernetzte Polyacrylsäure vom Typ A (Carbomer; Viskosität [0,5%] = 4000 - 11000 mPas; ungefähre relative Molmasse: 1250000) (Funktion: Quellmittel), Polyvinylpyrrolidon (PVP) des Typs Kollidon^{®} K25 (Funktion: Bindemittel) und Talkum (Funktion: Trennmittel) besprüht. Auf diese Weise erfolgt die Beladung mit der Quellschicht. Isopropylalkohol wird dabei als Lösungsmittel verwendet. Der Feststoffanteil in der Suspension beträgt ca. 11% w/w.
- Retardschichtpellets:
   Im nächsten Schritt werden die Pellets mit der die Wirkstofffreisetzung modifizierenden Polymerkombination befilmt. Dabei wird eine Kombination aus Ammoniummethacrylat-Copolymer Typ B (Poly(meth)acrylsäure-methyl/ethyl/2-trimethylaminoethylester-Copolymerisat mit einem Verhältnis von Ethylacrylat zu Methylmethacrylat und Trimethylammonioethylmethacrylat von 1:2:0,1 = Eudragit^{®} RS 12,5) und Ammoniummethacrylat-Copolymer Typ A (Poly(meth)acrylsäure-methyl/ethyl/2-trimethylaminoethylester-Copolymerisat mit einem Verhältnis von Ethylacrylat zu Methylmethacrylat und Trimethylammonioethylmethacrylat von 1:2:0,2 = Eudragit^{®} RL 12,5) verwendet. Beide Polymere bilden wasserunlösliche aber permeable Filme, die in einer 12,5% w/w Lösung in Isopropylalkohol (60% w/w) und Aceton (40% w/w) mit Natriumlaurylsulfat als Benetzungsmittel der Sprühlösung bzw. -suspension zugesetzt werden. Die Sprühlösung bzw. -suspension enthält zusätzlich noch Triethylcitrat (Funktion: Weichmacher) und Talkum (Funktion: Trennmittel). Der Feststoffanteil der organisch-wässrigen Sprühlösung bzw. -suspension beträgt ca. 14% w/w. Die Bestandteile der Retardschicht sind in dieser Sprühlösung bzw. -suspension, die aus Isopropylalkohol (ca. 88% w/w) und Wasser (ca. 12% w/w) besteht, suspendiert. Nach dem Befilmen und Trocknen der Pellets liegt die Retardschicht direkt auf der Quellschicht.
- Magensaftresistente Pellets:
   Im letzten Schritt werden die Pellets magensaftresistent überzogen. Dazu wird eine organisch-wässrige Lösung von Eudragit^{®} L (Polymethacrylsäure/Polymethylmethacrylat-Copolymerisat mit einem Verhältnis von Polymethacrylsäure zu Polymethylmethacrylat von 1:1) eingesetzt. Das Polymer bildet einen darmsaftlöslichen Überzug. Neben dem anionischen Polymer enthält die Sprühlösung bzw. -suspension noch Triethylcitrat (Funktion: Weichmacher) und Talkum (Funktion: Trennmittel), so dass sich ein Feststoffanteil von ca. 15% ergibt. Das Lösungsmittel besteht aus 85% w/w Isopropylalkohol und 15% w/w Wasser. Das Beladen der Pellets mit dieser Schicht stellt sicher, dass keine Wirkstofffreisetzung im Magen erfolgt.

Das Überziehen der Pellets mit den einzelnen Schichten erfolgt bevorzugt in Wirbelschichtanlagen. Da die einzelnen Schichten als Flüssigkeiten bzw. Suspensionen aufgebracht werden, muss die als Träger verwendete flüssige Phase entfernt werden. Dies geschieht durch eine geeignete Belüftung, wobei die Umgebungstemperatur moderat erhöht werden kann, allerdings nur bis zu einer solchen Temperatur, dass keine unerwünschten Nebenreaktionen erfolgen.

Die gewünschte Funktionalität der Mehrschichtpellets ergibt sich durch das optimale, sequentielle Auflösen und Quellen der aufgetragenen Schichten auf dem Weg durch den Verdauungstrakt: Sobald die Pellets den Dünndarm erreichen, löst sich zunächst das Polymer der äußersten Schicht (e) auf. Die intestinale Verdauungsflüssigkeit dringt durch die permeable Retardschicht (d) in das Innere der Pellets. Die pH-Verhältnisse des Dünndarms (pH < 5.5 - ca. 7,2) führen zu einer Quellung der Polyacrylsäure, dem Polymer der Quellschicht. Die damit verbundene Volumenvergrößerung erhöht den Innendruck auf die Retardschicht, wodurch sich kontinuierliche Fehlstellen in dieser Schicht ausbilden. Auf diese Weise entstehen Öffnungen, durch die das in der Verdauungsflüssigkeit gelöste Budesonid nach außen diffundieren kann. Auf diese Weise kann der in der Verdauungsflüssigkeit gelöste Wirkstoff über einen langen Zeitraum nach außen diffundieren und so am Wirkort freigesetzt werden und lokal wirken. Dieser Prozess verläuft zeitabhängig und startet in vollem Umfang, wenn die Pellets das Kolon erreicht haben.

Eine Dosis der so entwickelten Mehrschichtpellets kann in Hartgelatinekapseln abgefüllt werden. Die qualitative und quantitative Zusammensetzung einer bevorzugten Kapsel mit einer Dosis von 9 mg Budesonid ist in **Tabelle 1** zusammengefasst. Die verwendeten Lösungsmittel sind dabei nicht Bestandteil der Zusammensetzung, da sie als flüchtige Bestandteile während des Prozesses herausgetrocknet werden.

Es wurden verschiedene Prototypen der bevorzugten Ausführungsform hergestellt, die im Wesentlichen eine Zusammenstellung, wie in Tabelle 1 näher dargestellt, aufweisen.

**Tabelle 1: Zusammensetzung von bevorzugten erfindungsgemäßen Budesonid-Mehrschichtpellets in einer Kapsel**

| **Zusammensetzung [mg]** | | |
|---|---|---|
| Bestandteil | Funktion | Menge per Kapsel |
| 1. Wirkstoffpellets | | |
| Budesonid | Arzneilich wirksamer Bestandteil | 9,00 mg |
| Laktose-Monohydrat | Füllmittel | 36,00 mg |
| Polyvinylpyrrolidon (Kollidon^{®} K25) | Bindemittel | 2,70 mg |
| Polyoxyethylen(20)-sorbitan-monooleat (Polysorbat 80; Tween 80) | Benetzungsmittel | 0,71 mg |
| Talkum | Trennmittel | 16,50 mg |
| Zucker-Stärkepellets | Starterpellets | 260,00 mg |
| Summe: | | 324,91 mg |

| 2. Quellschichtpellets | | |
|---|---|---|
| Carbomer | Quellmittel | 24,00 mg |
| Polyvinylpyrrolidon (Kollidon^{®} K25) | Bindemittel | 18,00 mg |
| Talkum | Trennmittel | 18,00 mg |
| Summe: | | 384,91 mg |

| 3. Retardschichtpellets | | |
|---|---|---|
| Eudragit^{®} RS 12,5 | Retardpolymer | 6,30 mg |
| Eudragit^{®} RL 12,5 | Retardpolymer | 2,70 mg |
| Trietylcitrat | Weichmacher | 0,90 mg |
| Talkum | Trennmittel | 7,20 mg |
| Summe: | | 402.01 mg |

| 4. Magensaftresistente Pellets | | |
|---|---|---|
| Eudragit^{®} L | Darmsaftlösliches Polymer | 16,00 mg |
| Triethylcitrat | Weichmacher | 1,60 mg |
| Talkum | Antiklebemittel | 8,00 mg |
| Summe: | | 428,81 mg |

| 5. Hartgelatinekapsel | | |
|---|---|---|
| Gelatine mit Titandioxid | Kapselhülle | 97,00 mg |
| Total: | | 525,81 mg |

Das *in vitro* Freisetzungsprofil der erfindungsgemäßen Pellets ist in **Beispiel 1** wiedergegeben. Die geforderten Kriterien von Magensaftresistenz und kontinuierlicher Freisetzung im künstlichen Darmsaft werden den Vorgaben entsprechend erfüllt. **Beispiel 2** zeigt, dass die gewünschte Freisetzung von Budesonid aus den Pellets nur durch die kombinierte Anwendung von Quell- und Retardschicht überraschend realisiert werden kann.Erst der für die erfindungsgemäße Anmeldung beschriebene sequentielle Auftrag von Quell- und Retardschicht auf die Wirkstoffpellets ermöglicht es, dass Budesonid an den Wirkort im Kolon verbracht werden kann.

### Negativbeispiel (Extrusionspellet, Beispiel 4)

Es wurde versucht, mit einer Technologievariante über Extrusion mit anschliessender Späronisierung eine pharmazeutischen Formulierung zu entwickeln, die das gewünschte Freisetzungsprofil zeigt.

Bei Extrusionspellets (Negativbeispiel) handelt es sich um Pulveragglomerate, wobei die Herstellung der Partikel durch Feucht- oder Schmelzextrusion einer Pulvermasse mit anschließendem Sphäronisieren erfolgt. Extrusionspellets wurden nur als Referenz verwendet. Extrusionspellets erlauben in der Regel eine höhere Wirkstoffbeladung als Mehrschichtpellets, was bei der vorliegenden Erfindung jedoch nicht von Relevanz ist, da topische Corticoide niedrigdosiert verabreicht werden. Für die Extrusion werden Schneckenextruder verwendet, wobei durch Anwendung von Druck eine feuchte oder geschmolzene Masse durch Öffnungen bestimmter Größe gepresst wird. Die so erhaltenen Stränge werden zu zylindrischen Pellets geschnitten und anschließend zu Kugeln ausgerundet. Die Pellets können ebenfalls in Wirbelschichtanlagen beschichtet werden. Die Herstellung von Extrusionspellets erfolgt im Gegensatz zu Mehrschichtpellets diskontinuierlich. Wesentlich für die Auswahl des Pellettyps sind unter anderem die physikalisch-chemischen Eigenschaften des zu verarbeitenden Wirkstoffs, die erforderliche Wirkstoffbeladung und der gewünschte Freisetzungsmechanismus, um ein gezieltes Freisetzungsprofil zu erhalten. Erfindungsgemäß werden keine Extrusionspellets verwendet.
• Der Aufbau der Extrusionspellets folgte dabei folgendem Schema bezüglich qualitativer Zusammensetzung und Herstellung:

| |
|---|
| Wirkstoffgranulat |
| ↓ |
| Extrusion |
| ↓ |
| Sphäronisierung |
| ↓ |
| Magensaftresistente Pellets |
| ↓ |
| Abfüllung in Hartqelatinekapseln oder Stickpacks |

• Wirkstoffgranulat:
Eine Pulvermischung aus Budesonid, Laktose-Monohydrat (Funktion: Füllmittel), Carrageenan (Gelcarin GP911 NF) (Funktion: Matrixbildner und Extrusionshilfsmittel) und Calciumchlorid (Funktion: Verstärkung der Gel- bzw- Matrixbildung) wurde wässrig granuliert. Die Granulierflüssigkeit enthielt noch Polyvinylacetat (Kollicoat SR 30D) (Funktion: Retardpolymer und Matrixbildner). Nach Sieben der feuchten Masse erfolgte die Extrusion.
• Extrusion und Sphäronisierung:
Die feuchte Masse wurde in einem Schneckenextruder bei 65°C durch eine Lochplatte (Matrize mit definierten Öffnungen, Ø 1,0 - 2,2 mm) gepresst und anschließend in zylinderförmige Formlinge geschnitten. Diese Formlinge werden bei max. 50°C ausgerundet, getrocknet und zuletzt der Feinanteil durch Sieben (Maschenweite: 1000 µm) abgetrennt.
• Magensaftresistente Pellets:
Im letzten Schritt werden die Rundlinge magensaftresistent überzogen. Dazu wird eine wässrige Suspension aus einer Mischung von Eudragit^{®} FS 30 D (Poly(met)acrylsäure-metylester-Copolymerisat) und Eudragit^{®} L 30 D 55 (Poly(met)accrylsäure-etylester-Copolymerisat) im Verhältnis von ca. 80% w/w und 20% w/w eingesetzt. Beide Polymere bilden einen darmsaftlöslichen Überzug. Die wässrige Suspension enthält daneben noch Triethylcitrat (Funktion: Weichmacher), Glycerolmonostearat (Funktion: Antiklebemittel) und die Benetzungsmittel Polyoxyethylen(20)-sorbitan-monooleat (Polysorbat^{®} 80; Tween 80) sowie Natriumlaurylsulfat, so dass sich ein Feststoffanteil von ca. 21% ergibt. Das Beladen der Extrusionspellets mit dieser Schicht stellt sicher, dass keine Wirkstofffreisetzung im Magen erfolgt.
• Eine Dosis der so entwickelten Extrusionspellets kann in Hartgelatinekapseln abgefüllt werden. Die qualitative und quantitative Zusammensetzung einer Kapsel mit einer Dosis von 9 mg Budesonid ist in **Tabelle 2** zusammengefasst. Die verwendeten Lösungsmittel sind dabei nicht Bestandteil der Zusammensetzung, da sie als flüchtige Bestandteile während des Prozesses herausgetrocknet werden.
• Die Formulierung wurde mit dem Ziel entwickelt, dass die Matrix der Pellets nach Erreichen des Dünndarms und dem Auflösen der Polymere der äußersten Sicht durch die eingedrungene Verdauungsflüssigkeit aufquillt. Auf diese Weise sollte ein allmähliches Erodieren der Partikel während der Passage der Darmabschnitte erfolgen und so zu einer kontinuierlichen Wirkstofffreisetzung führen.

Das *in vivo* Freisetzungsprofil der Extrusionspellets ist in **Beispiel 4** wiedergegeben. Die geforderten Kriterien für eine gezielte, topische Anwendung im Kolon werden von den Extrusionspellets nicht erfüllt. Dieser Entwicklungsansatz führt also nicht zu dem gewünschten "Kolon-Targeting".

**Tabelle 2: Zusammensetzung der Budesonid-Extrusionspellets enthaltenden Kapsel (Negativbeispiel)**

| **Zusammensetzung [mg]** | | |
|---|---|---|
| Bestandteil | Funktion | Menge per Kapsel |
| 1. Wirkstoffgranulat | | |
| Budesonid | Arzneilich wirksamer Bestandteil | 9,00 mg |
| Polyvinylacetat (Kollicoat SR 30D) | Retardpolymer, Matrixbildner | 52,50 mg |
| Lactose-Monohydrat | Bindemittel | 28,50 mg |
| Carragenan (Gelcarin GP911 NF) | Matrixbildner, Extrusionshilfsmittel | 240,00 mg |
| Calciumchlorid | Gel- und Matrixbildner | 20,00 mg |
| Summe: | | 350,00 mg |

| 2. Magensaftresistente Pellets | | |
|---|---|---|
| Eudragit^{®} FS 30 D | Darmsaftlösliches Polymer | 48,00 mg |
| Eudragit^{®} L 30 D 55 | Darmsaftlösliches Polymer | 12,00 mg |
| Triethylcitrat (Citrofol AL) | Weichmacher | 2,80 mg |
| Glycerolmonostearat | Antiklebemittel | 3,10 mg |
| Polyoxyethylen(20)-sorbitan-monooleat (Polysorbat^{®} 80; Tween 80) | Benetzungsmittel | 1,10 mg |
| Summe: | | 417,00 mg |

| 3. Hartgelatinekapsel | | |
|---|---|---|
| Gelatine mit Titandioxid | Kapselhülle | 97,00 mg |
| Total: | | 514,00 mg |

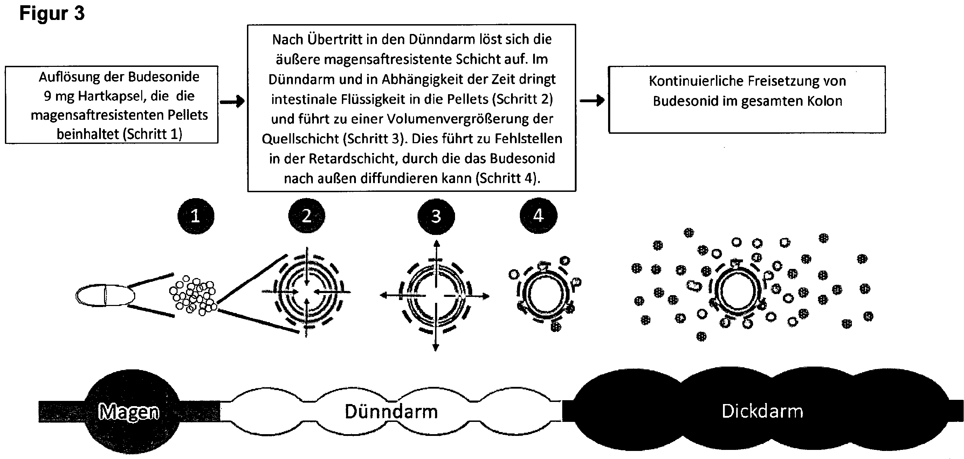

Die Auflösung der erfindungsgemäßen Formulierung ist schematisch in Figur 3 dargestellt.

Gegenüber dem negativen Vergleichsbeispiel zeigten die erfindungsgemäßen Mehrschichtpellets völlig andere Blutspiegel. Nach dem Auflösen des magensaftresistenten Films wird hier der bereits beschriebene komplexe Freisetzungsmechanismus ausgelöst. Die Plasmaspiegel von Budesonid fluten nicht an, sondern zeigen einen modifizierten, verzögerten und prolongierten Verlauf über mehrere Stunden.

Die Ergebnisse der Pharmakokinetikstudie bestätigen die *in vitro*-Ergebnisse in Beispiel 1 und demonstrieren eindrucksvoll, dass der Wirkstoff über die gesamte Passagezeit des Dickdarms aus den Mehrschichtpellets freigesetzt wird und daher an den Stellen ankommt, wo er seine Wirkung an der Schleimhaut des Kolons entfalten kann.

In einer offenen, monozentrischen, randomisierten Pharmakokinetikstudie wurden die erfindungsgemäßen Mehrschichtpellets mit Budenofalk 3 mg Hartkapseln aus dem Stand der Technik verglichen. Die mittleren Plasmakonzentrationen des Wirkstoffs wurden nach oraler Einmalgabe einer Dosis von 9 mg über die Zeit gemessen, wobei die Bioverfügbarkeit von Budesonid aus der Referenzformulierung bekannt ist. Beispiel 3 zeigt die Ergebnisse der vergleichenden Pharmakokinetikstudie.

Wie den Plasmaspiegeln in **Beispiel 3** zu entnehmen ist, ist die im Stand der Technik beschriebene Formulierung nicht geeignet, Budesonid gezielt in das Kolon zu transportieren. Der Blutspiegel von Budesonid steigt rasch und stark an. Dieser Verlauf ist für ein Kolon-Targeting unerwünscht, da der Wirkstoff nicht in ausreichender Konzentration am Wirkort ankommt, sondern bereits vorher zu einem großen Anteil in den oberen Dünndarmabschnitten resorbiert wird und damit für eine lokale bzw. topische Wirksamkeit im Dickdarm nicht mehr zur Verfügung steht. Dieses *in vivo*-Verhalten lässt sich mit der Zusammensetzung der Formulierung erklären. Die im Stand der Technik beschriebenen Budenofalk 3 mg Hartkapseln enthalten lediglich magensaftresistent-überzogene Budesonidpellets. Nach der Magenpassage löst sich dieser Überzug mit einer gewissen Verzögerungszeit auf und der Wirkstoff wird dann sofort und vollständig freigesetzt. Das Kolon wird auf diese Weise nicht erreicht. Auch die Ergebnisse einer klinischen Studie, bei der diese im Stand der Technik beschriebene Formulierung getestet wurde, zeigen, dass Budenofalk 3 mg Hartkapseln (beschrieben in WO 95/08323) zur Behandlung der ulzerativen Colitis nicht geeignet sind. Eine zumindest mit einer Mesalazintherapie vergleichbare Wirksamkeit in der Behandlung von Patienten mit leichter bis moderarter Colitis ulcerosa konnte nicht gezeigt werden (Gross et al., 2011).

Die Wirksamkeit und Verträglichkeit der neuen Mehrschichtpellets wurden auch in einer offenen, klinischen Studie untersucht. Wie in **Beispiel 5** beschrieben, belegen die Ergebnisse eindrucksvoll die klinische Wirksamkeit der Pellets. Die Ergebnisse demonstrieren, dass die erfindungsgemäßen Pellets den Wirkstoff an den gewünschten Zielort bringen und Patienten erfolgreich behandelt werden können, die an einer Colitis ulcerosa erkrankt sind, und die mit Mesalazin **nicht** ausreichend behandelt werden konnten (Mesalazin-refraktär).

Im Gegensatz zu den Mehrschichtpellets gleicht der in der Pharmakokinetikstudie in **Beispiel 4** erhaltene Blutspiegelverlauf der Extrusionspellets (Negativbeispiel) mehr dem Stand der Technik als dem gewünschten Zielprofil einer im Kolon freisetzenden Formulierung. Die Budesonidspiegel fluten sogar noch schneller an als bei der Referenzformulierung und fallen auch entsprechend schnell wieder ab, so dass die Wirkstofffreisetzung vor Erreichen des Kolons abgeschlossen ist und offensichtlich keine nennenswerten Mengen an Budesonid den Dickdarm erreichen.

Wesentliche Aspekte der vorliegenden Erfindung werden in den anliegenden Figuren gezeigt und erläutert:
Figur 1 stellt eine schematische Darstellung des Verdauungstraktes dar.
Figur 2 zeigt schematisch den Aufbau der erfindungsgemäßen Pellets zur verzögerten Freisetzung des Wirkstoffes.
In Figur 3 wird die Wirkungsweise der erfindungsgemäßen Pellets in Abhängigkeit von der jeweiligen Region im Verdauungstrakt dargestellt.
Figur 4 zeigt die *in-vitro* Freisetzungsprofile verschiedener Pellets, wobei Wirkstoffpellets, Quellschichtpellets, Retardschichtpellets und magensaftresistente Pellets miteinander hinsichtlich der Freisetzung verglichen werden, wobei die erfindungsgemäße Formulierung als magensaftresistente Pellets bezeichnet ist. Die Bedingungen sind in Beispiel 1 näher erläutert. SGF bedeutet künstlicher Magensaft; SIF bedeutet künstlicher Darmsaft.
Figur 5 zeigt das *in-vitro* Freisetzungsprofil von erfindungsgemäßen Pellets mit und ohne Quellschicht. Die Bedingungen des Versuchs sind in Beispiel 2 näher erläutert. Die Figur 5 belegt, dass eine Retardschicht ohne darunterliegende Quellschicht keine Freisetzung des Wirkstoffes ermöglicht.
Figur 6 zeigt die *in-vivo* Freisetzungs-Charakteristik, die in offenen, randomisierten Pharmakokinetik-Studien erhalten wurde. Es handelt sich hierbei um *in vivo*-Daten, was etwaige Unterschiede zu *in vitro*-Daten erklärt.
Figur 7 belegt, dass mit Hilfe der erfindungsgemäßen Formulierungen tatsächlich das gesamte Colon effektiv behandelt werden kann. In Beispiel 6 wurde die Wirksamkeit und Verträglichkeit der erfindungsgemäßen Budesonid-Pellets untersucht, wobei Figur 7 den Anteil der Patienten mit klinischer Remission zeigt in Abhängigkeit von der Lokalisation der jeweiligen Erkrankung.
Figur 8 zeigt die Wirksamkeit der erfindungsgemäßen Formulierungen, gemessen an der Anzahl der blutigen Stühle pro Woche. Die Figur 8 belegt, dass die Anzahl der blutigen Stühle pro Woche im Lauf der Behandlungszeit effektiv gesenkt werden konnte.

Unter Verwendung der vorstehend beschriebenen Materialien und Methoden wurden verschiedene *in vivo Pharmakokinetik-Profile* gemessen. Die nachfolgenden Beispiele verdeutlichen die Erfindung.

### Beispiel 1: In vitro Freisetzungsversuche der Budesonid-Mehrschichtpellets (erfindungsgemäße Formulierung)

Um den Effekt sowie den Einfluss der einzelnen Schichten auf die Wirkstofffreisetzung zu zeigen, wurden die *in vitro*-Versuche mit Wirkstoffpellets, Quellschichtpellets, Retardschichtpellets und magensaftresistenten Pellets aus einer Charge durchgeführt. Die Prüfung erfolgte dabei mit einer Menge an Pellets, die einer Dosis von 9 mg Budesonid entspricht. Die äußere Hartgelatinekapsel hat keinen Einfluss auf die Wirkstofffreisetzung der Pellets und wurde daher bei diesen Versuchen weggelassen.

Die *in vitro*-Freisetzungsversuche der Pellets umfassten dabei eine zweistufige Prüfung. Im ersten Schritt erfolgte die Prüfung der Pellets im künstlichen Magensaft (pH 1,2) über eine Dauer von zwei Stunden mit anschließendem Transfer der Prüfmuster in künstlichen Darmsaft (pH 6,5) sowie einer weiteren Prüfung der Wirkstofffreisetzung über 7 Stunden. Zur Gewährleistung von Sinkbedingungen enthielten beide Medien 0,1% Polysorbat 80 als Netzmittel. Die Parameter der Freisetzungsprüfung waren dabei wie folgt:

| | |
|---|---|
| • Freisetzungsapparatur: | Apparatur 2 des europäischen Arzneibuchs beschrieben in Kapitel 2.9.3 (Rührblattmethode) |
| • Volumen des Freisetzungsmediums: | 900 ml künstlicher Magensaft (SGF) |
| | 900 ml künstlicher Darmsaft (SIF) |
| • Umdrehungsgeschwindigkeit: | 75 Umdrehungen pro Minute |
| • Temperatur; | 37,0°C ± 0,5°C |
| • Prüfmedien: | |
| - Künstlicher Magensaft (SGF): | 0,1 N HCl mit 0,1% Polysorbat 80, pH 1,2 |
| - Künstlicher Darmsaft (SIF): | Phosphatpuffer (KH₂PO₄, NaCl, NaOH) mit 0,1% Polysorbat, pH 6,5 |
| • Charge der Prüfmuster: | 180013457 |
| • Anzahl Prüfmuster: | N = 6 |
| • Probenzug: | |
| - Wirkstoffpellets: | 5, 10, 15, 30, 45, 60 und 120 Minuten im künstlichen Magensaft |
| - Quellschichtpellets: | 5, 10, 15, 30, 45, 60 und 120 Minuten im künstlichen Magensaft sowie |
| | 15, 30, 45, 60, 90 und 180 Minuten im künstlichen Darmsaft |
| - Retardschichtpellets: | 15, 30, 45, 60, 90 und 120 Minuten im künstlichen Magensaft |
| | 30, 90, 150, 210, 270, 330, 390 und 420 Minuten im künstlichen Darmsaft |
| Magensaftresistente Pellets: | 15, 30, 45, 60, 90 und 120 Minuten im künstlichen Magensaft |
| | 30, 90, 150, 210, 270, 330, 390 und 420 Minuten im künstlichen Darmsaft |
| • Gehaltsbestimmung Budesonid: | HPLC/UV (λ = 255 nm) |

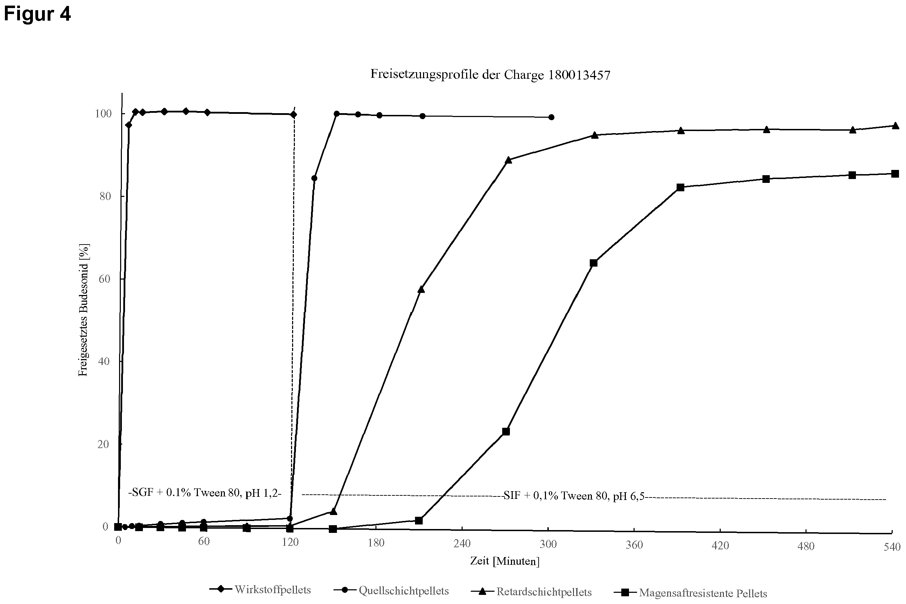

Die Figur 4 zeigt die *in-vitro* Freisetzungsprofile der einzelnen Pellets. Die Wirkstoffpellets (ohne Überzug) setzen bereits vollständig im Magensaft frei. Nach 5 Minuten sind mehr als 85% des Budesonids gelöst. Die Quellschichtpellets zeigen eine geringe Freisetzung innerhalb der zweistündigen Prüfung im Magensaft (< 5%) und setzten nach dem Transfer in Darmsaftmedium innerhalb von 15 Minuten vollständig frei (≥ 85%). Dieses Verhalten spiegelt die physikalisch-chemischen Eigenschaften der verwendeten Polyacrylsäure (Carbomer) wider, die auf Grund ihres pKs-Wertes von ca. 6 in Abhängigkeit vom pH-Wert des Mediums entweder unlöslich, quellbar oder löslich ist. Unter den Bedingungen von künstlichem Magensaft (pH 1,2) liegt das hochmolekulare Polymer der Acrylsäure protoniert vor und ist unlöslich. Die Quellschicht verbleibt auf den Pellets. Nach dem Transfer in den Darmsaftpuffer steigt der pH-Wert in den Bereich des pKs-Wertes und führt zu einer teilweisen Deprotonierung der Carbonsäuren. Dadurch können Wassermoleküle ins enge Polymergerüst eingelagert werden, was zum Aufbau eines Gelgerüsts und damit zur Quellung führt. Ohne die Retardschicht kommt dieser Effekt der Quellschicht aber nicht zur Geltung und Budesonid wird rasch freigesetzt.

Erst nach dem Auftragen der Retardschicht auf die Quellschichtpellets kann sich der oben beschriebene Effekt innerhalb der Pellets voll entfalten (siehe Figur 4: Freisetzungsprofil der Retardschichtpellets). Das Freisetzungsprofil der Retardschichtpellets im Darmsaft ändert sich deutlich und geht in die gewünschte sigmoide Form verbunden mit einer verzögerten Budesonid-Freisetzung über. Nach ca. 120 Minuten im Magensaft wird die vollständige Wirkstofffreisetzung aus solchen Retardschichtpellets im Darmsaft nach weiteren ca. 150 Minuten erreicht. Eine vorzeitige Freisetzung im Magensaft tritt also nicht ein.

Aber erst die endgültige Formulierung mit einer zusätzlichen magensaftresistenten Beschichtung zeigt schließlich das für ein Kolon-Targeting gewünschte Freisetzungsprofil der erfindungsgemäßen Formulierung. Die Freisetzung von Budesonid startet ca. 90 Minuten nach der Magenpassage. Eine vorzeitige Freisetzung erfolgt also nicht. Nach dieser Zeit haben die Pellets den Dünndarm passiert und das Kolon erreicht. Anschließend wird der Wirkstoff mit sigmoidaler Kinetik über mindestens sieben Stunden kontinuierlich freigesetzt. Zusammengefasst erfolgt die Freisetzung der erfindungsgemäßen Formulierung in diesem *in vitro*-System nach folgenden Kriterien:
- SGF ("simulated gastric fluid" entsprechend künstlicher Magensaft, pH 1,2:
   Nach 2 Stunden: Keine Freisetzung=Magensaftresistenz
- SIF ("simulated intestinal fluid" entsprechend künstlicher Darmsaft), pH 6,5:
   Nach 270 min: 10-30% Freisetzung
   Nach 330 Minuten: 40-70% Freisetzung
   Nach 540 Minuten: ≥ 80% Freisetzung

Die Ergebnisse der Gehaltsbestimmung der Wirkstoff-, Quellschicht-, Retardschicht- und magensaftresistenten Pellets mit HPLC/UV belegen, dass die in den einzelnen *in vitro* Freisetzungsuntersuchungen getesteten Pellets mit der erforderlichen Budesonidmenge von 9 mg beladen waren (entsprechend dem Inhalt einer Kapsel).

| | |
|---|---|
| • Wirkstoffpellets: | 100,0% |
| • Quellschichtpellets: | 101,9% |
| • Retardschichtpellets: | 101,6% |
| • Magensaftresistente Pellets: | 100,3% |

### Beispiel 2: In vitro Freisetzungsversuche von Budesonid-Retardschichtpellets mit und ohne Quellschicht

Um das Zusammenspiel von Quellschicht und Retardschicht bei der Wirkstofffreisetzung der erfindungsgemäßen Anmeldung zu zeigen, wurden Budesonid-Retardschichtpellets mit und ohne Quellschicht nach folgender Zusammensetzung hergestellt:

| **Zusammensetzung [mg]** | | |
|---|---|---|
| Bestandteil | Menge per Dosis von 9 mg Budesonid | |
| | Charge R020 | Charge R029 |
| 1. Wirkstoffpellets | | |
| Budesonid | 9,00 mg | 9,00 mg |
| Laktose-Monohyd rat | 36,00 mg | 36,00 mg |
| Polyvinylpyrrolidon (Kollidon K25) | 2,70 mg | 2,70 mg |
| Polyoxyethylen(20)-sorbitan-monooleat (Polysorbat 80, Tween 80) | 0,71 mg | 0,71 mg |
| Talkum | 16,50 mg | 16,50 mg |
| Zucker-Stärkepellets | 260,00 mg | 260,00 mg |
| Summe: | 324,91 mg | 324,91 mg |

| 2. Quellschichtpellets | | |
|---|---|---|
| Carbomer | --- | 24,00 mg |
| Polyvinylpyrrolidon (Kollidon K25) | --- | 18,00 mg |
| Talkum | --- | 18,00 mg |
| Summe: | 324,91 mg | 384,91 mg |

| 3. Retardschichtpellets | | |
|---|---|---|
| Eudragit^{®} RS 12,5 | 10,50 mg | 10,50 mg |
| Eudragit^{®} RL 12,5 | 4,50 mg | 4,50 mg |
| Trietylcitrat | 1,50 mg | 1,50 mg |
| Talkum | 12,0 mg | 12,0 mg |
| Summe: | 353,41 mg | 413,41 mg |

Hinsichtlich der Zusammensetzung der Wirkstoff- und Quellschicht entspricht die Charge R029 der erfindungsgemäßen Anmeldung. Im Vergleich zur erfindungsgemäßen Anmeldung wurde jedoch der Auftrag der Retardschicht absichtlich um den Faktor 1,7 erhöht. Mit dieser Testcharge sollte gezeigt werden, dass die Wechselwirkung bzw. das Zusammenspiel beider Schichten im Rahmen der Wirkstofffreisetzung auch unter extremen Bedingungen funktioniert und damit ein robuster Effekt der Quellschicht existiert. Die Charge R020 enthielt keine Quellschicht und diente als Kontrollcharge. Mit Ausnahme dieses Unterschieds waren Kontroll- und Testcharge identisch zusammengesetzt. Die Pellets beider Chargen wurden nicht mit einem magensaftresistenten Film überzogen. Die Parameter der Freisetzungsprüfung waren wie folgt:

| | |
|---|---|
| • Freisetzungsapparatur: | Apparatur 2 des europäischen Arzneibuchs beschrieben in Kapitel 2.9.3 (Rührblattmethode) |
| • Volumen des Freisetzungsmediums: | 900 ml künstlicher Darmsaft (d.h. eine Prüfung auf Magensaftresistenz erfolgte nicht) |
| • Umdrehungsgeschwindigkeit: | 75 Umdrehungen pro Minute |
| • Temperatur; | 37,0°C ± 0,5°C |
| • Prüfmedium: | |
| - Künstlicher Darmsaft (SGF): | Phosphatpuffer (KH₂PO₄, NaCl, NaOH) mit 0,1% Polysorbat, pH 6,5 |
| • Chargen: | |
| - Testcharge: | R029 |
| - Kontrollcharge: | R020 |
| • Anzahl Prüfmuster: | N = 2 |
| • Probenzug und Gehaltsbestimmung: | Kontinuierlich mit UV-On-Line-Messung (λ = 255 nm) |

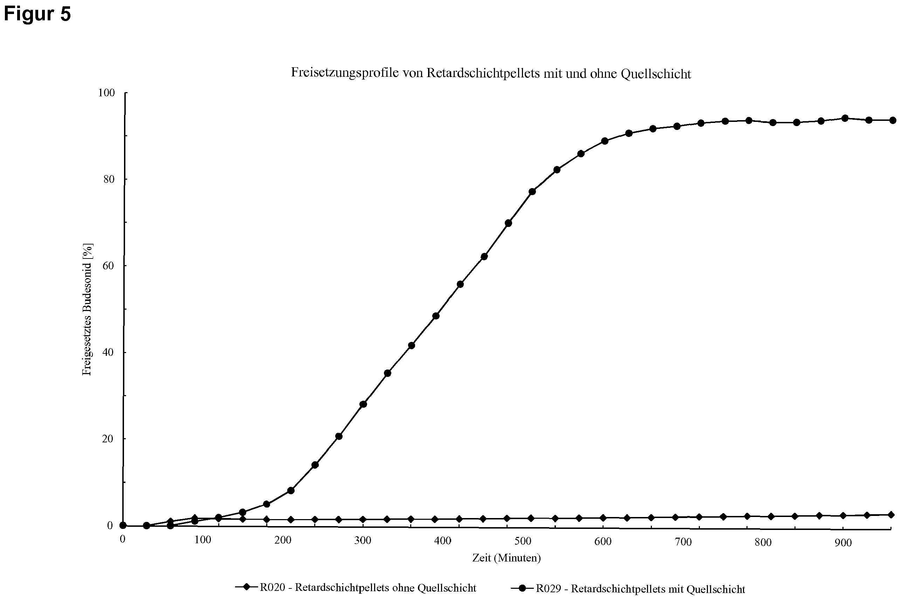

Die Figur 5 zeigt die *in-vitro* Freisetzungsprofile von Test- und Kontrollcharge. Der Effekt der Quellschicht ist beindruckend und völlig überraschend. Während aus der Kontrollcharge über Stunden keinerlei Budesonid freigesetzt wurde, entspricht das Freisetzungsprofil der Testcharge dem Profil der erfindungsgemäßen Anmeldung. Somit ermöglicht nur die gleichzeitige Anwesenheit von Quell- und Retardschicht die gewünschte verzögerte Freisetzung von Budesonid aus den Pellets. Die Retardschicht alleine ist diesbezüglich nicht funktionsfähig. Somit ist nur die für die erfindungsgemäße Anmeldung beschriebene Kombination aus Quell- und Retardschicht in der Lage, Budesonid an seinen Wirkort zu verbringen, so dass beide Schichten zusammen als die Wirkstofffreisetzung kontrollierenden Bestandteile der Formulierung betrachten werden müssen.

### Beispiel 3: In vivo Pharmakokinetikprofile der Budesonid-Mehrschichtpellets enthaltenden Kapsel (erfindungsgemäße Formulierung) und der Budesonidkapsel im Stand der Technik/Referenz

In einer offenen, randomisierten Phase I-Studie zur Pharmakokinetik der erfindungsgemäßen Formulierung wurde jeweils 12 gesunden, männlichen Probanden im nüchternen Zustand eine Einmaldosis der erfindungsgemäßen Formulierung (BUX-PVII; 9mg Budesonid) und der Budesonidkapsel im Stand der Technik (3x3mg Budesonid als Einmalgabe in Form der Referenz-Pellets) verabreicht. Wesentliche pharmakokinetische Parameter sind in der folgenden Tabelle 3 dargestellt.

**Tabelle 3:**

| | Behandlung mit erfindungsgemäßen Pellets (BUX-PVII) | | Behandlung mit Referenz-Pellets (3×3mg Budesonid) | |
|---|---|---|---|---|
| | Cₘₐₓ (ng/mL) | tmax(h) | Cₘₐₓ (ng/mL) | tmax(h) |
| N | 12 | 12 | 12 | 12 |
| Mean (SD) | 0.928 (0.755) | 7.75 (3.71) | 4.60 (4.85) | 5.26 (0.81) |
| Min | 0.193 | 2.00 | 1.04 | 4.50 |
| Max | 2.62 | 13.00 | 16.1 | 6.50 |

In der Tabelle 3 bedeutet N = Anzahl der Probanden; Mean (SD) bedeutet Durchschnittswert/Standardabweichung; Min = Minimalwert; Max = Maximalwert. Bei der Tabelle 3 sind für Cₘₐₓ und tₘₐₓ der jeweilige Mittelwert der maximalen Budesonidplasmaspiegel der einzelnen Patienten unabhängig vom Messzeitpunkt dargestellt.

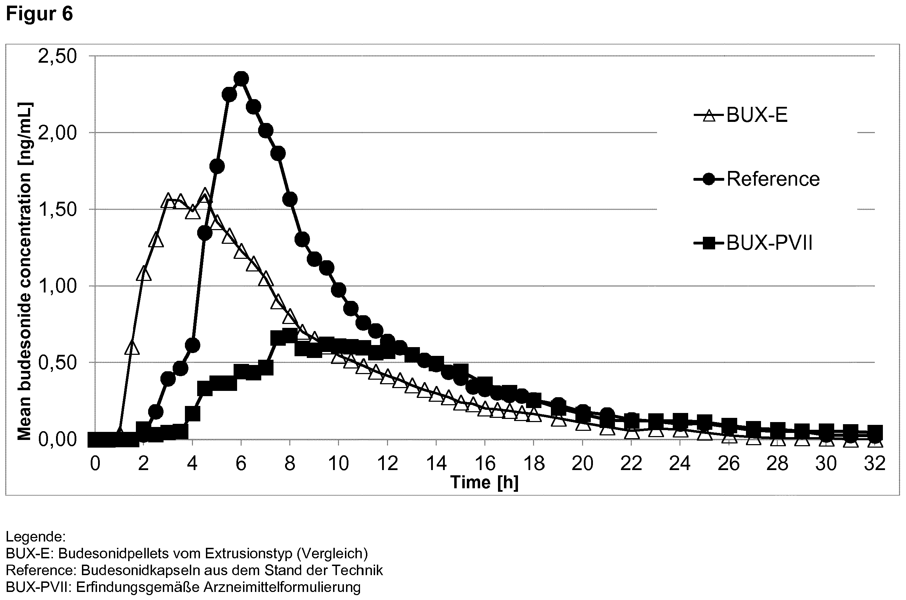

Die Ergebnisse der *in vivo* Pharmakokinetikstudie sind graphisch in Figur 6 dargestellt. Im Vergleich zu den Werten in Tabelle 3 sind in Figur 6 die Einzelwerte für den jeweiligen Zeitpunkt gemessen worden und der zugehörige Mittelwert errechnet worden. Daher unterscheiden sich die Werte der Tabelle 3 von den Werten der Figur 6.

### Beispiel 4: In vivo Pharmakokinetikprofile der Budesonid-Extrusionspellets enthaltenden Kapsel (Negativbeispiel)

In einer offenen, randomisierten Phase I-Studie zur Pharmakokinetik von Budesonid-Extrusionspellets wurde 16 gesunden, männlichen Probanden im nüchternen Zustand jeweils eine Einmaldosis der Budesonid-Extrusionspellets (9mg in einer Gelatinekapsel) als Einmalgabe verabreicht. Wesentliche pharmakokinetische Parameter sind in der folgenden Tabelle 4 dargestellt. Auch in der Tabelle 4 sind für Cₘₐₓ und tₘₐₓ der jeweilige Mittelwert der maximalen Budesonidplasmaspiegel der einzelnen Patienten unabhängig vom Messzeitpunkt dargestellt.

**Tabelle 4:**

| | Behandlung mit Extrusions-Budesonidpellets (BUX-E) | |
|---|---|---|
| | Cₘₐₓ (ng/mL) | tₘₐₓ(h) |
| N | 16 | 16 |
| Mean (SD) | 2.478 (0.9683) | 3.97 (1.407) |
| Min | 0.913 | 2.00 |
| Max | 3.95 | 7.00 |

Die Tabellen 3 und 4 belegen, dass nur mit den erfindungsgemäßen Pellets die gewünschte späte Freisetzung (tₘₐₓ = 7.75) erzielt werden konnte, wohingegen beim Stand der Technik tₘₐₓ = 5.26 und bei den Extrusionspellets tₘₐₓ = 3.97 beobachtet wurde.

### Beispiel 5:

Die Ergebnisse dieses Beispiels sind in Figur 6 graphisch dargestellt.Figur 6 zeigt die Budesonidplasmaspiegel über die Zeit aus den offenen, monozentrischen, randomisierten Pharmakokinetikstudien. Dargestellt sind die jeweiligen Mittelwerte der Budesonidplasmaspiegel aller Patienten zum jeweiligen Messzeitpunkt. Demgegenüber spielt es bei den Werten der Tabelle 3 bzw. 4 keine Rolle, wann der jeweilige Patient den entsprechenden Cₘₐₓ- bzw. tₘₐₓ-Wert zeigte. Neben der erfindungsgemäßen Formulierung wurden weitere Prototypen von budesonidhaltigen Arzneimittelformulierung getestet. Die in Figur 2 schematisch dargestellte Formulierung mit der Bezeichnung BUX-PVII erlaubt optimales Kolon-Targeting. Das zu erreichende *in-vivo* Freisetzungsprofil soll nach einer Verzögerungsphase - gleichmäßig über einen längeren Zeitraum bei gleichzeitig niedrigen Serumplasmaspiegeln - den Wirkstoff freisetzen. Die mittleren Plasmakonzentrationen für Budesonid nach Einmalgabe wurden über die Zeit gemessen. In der Figur 6 sind die Ergebnisse für zwei Referenzformulierungen (BUX-E und Reference) und für die erfindungsgemäße Formulierung (BUX-PVII) aufgetragen.

Lediglich die erfindungsgemäße Formulierung zeigt das gewünschte Freisetzungsprofil: Eine deutliche Verzögerung des Starts der Freisetzung (ca. 3 h), plateau-ähnliche Budesonidplasmaspiegel über einen langen Zeitraum (über ca. 8 h) und im Vergleich deutlich niedrigere Budesonidplasmaspiegel.

In dieser Studie waren die Profile der Plasmakonzentrationen über die Zeit für die erfindungsgemäße Arzneimittelformulierung homogener mit weniger Streuung zwischen den einzelnen Teilnehmern an der klinischen Studie.

### Beispiel 6:

In einer offenen, klinischen Studie über 8 Wochen wurde die Wirksamkeit und die Verträglichkeit eines Prototyps der neuen oralen 9 mg Budesonidformulierung untersucht, die täglich 1x eingenommen wurde. Ziel der Studie war es, eine klinische Remission, definiert über einen Aktivitätsscore der Erkrankung von ≤ 4 (Colitis Activity Index, CAI), und über die CAI-Subscores 1 und 2 von jeweils 0 zu erreichen. Der CAI Subscore 1 mit einem Wert 0 beschreibt eine Stuhlfrequenz von weniger als 18 pro Woche und der CAI Subscore 2 mit einem Wert 0 beschreibt, dass kein oder maximal 1 Stuhl pro Woche blutig sein darf. In dieser Studie wurden Patienten mit einer aktiven Colitis ulcerosa behandelt, die mit einem mesalazin-haltigen Präparat als Standardtherapie nicht erfolgreich behandelt werden konnten. Diese Vorbehandlung musste vor Start der klinischen Studie vor der ersten Einnahme der Studienmedikation beendet werden.

Insgesamt wurden 61 Patienten behandelt, von denen 52 die Studie protokollgemäß beendet haben. Der Anteil der Patienten, die das Ziel der Studie, den primären Endpunkt erreichten, ist in Tabelle 3 dargestellt. In der sogenannten FAS-Gruppe sind alle Patienten zusammengefasst, die mindestens 1x mit dem Prüfpräparat behandelt wurden. In der sogenannten PP-Gruppe diejenigen Patienten, die die Studie protokollgemäß abgeschlossen haben. In beiden Analysegruppen konnten Remissionsraten in dieser Mesalazin-refraktären Studienpopulation von etwa 50% erreicht werden.

**Table 5: Analyse des primären Endpunktes**

| **Analysegruppe** | **Zahl** (%) **der Patienten in klinischer Remission Typ 1 am Ende der Studie** | | | |
|---|---|---|---|---|
| | Total | | | Clopper-Pearson 95% **CI¹** |
| | N | **n** | % | |
| FAS | 61 | 29 | 47.5 | [34.60%, 60.73%] |
| PP 52 | | 28 | 53.8 | [39.47%, 67.77%] |

| | | | | |
|---|---|---|---|---|
| ¹CI, Confidence Intervall | | | | |

### (Full Analysis Set, FAS: Per Protocol Set, PP)

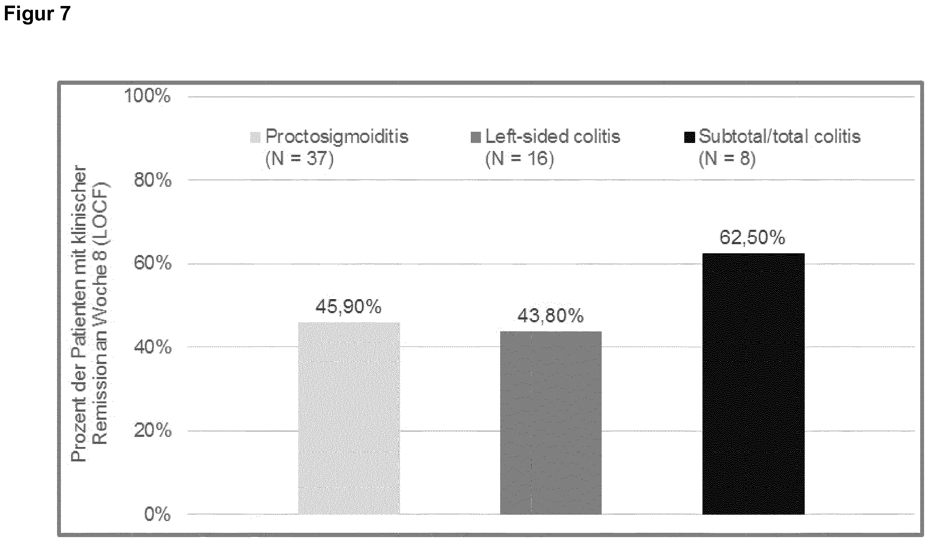

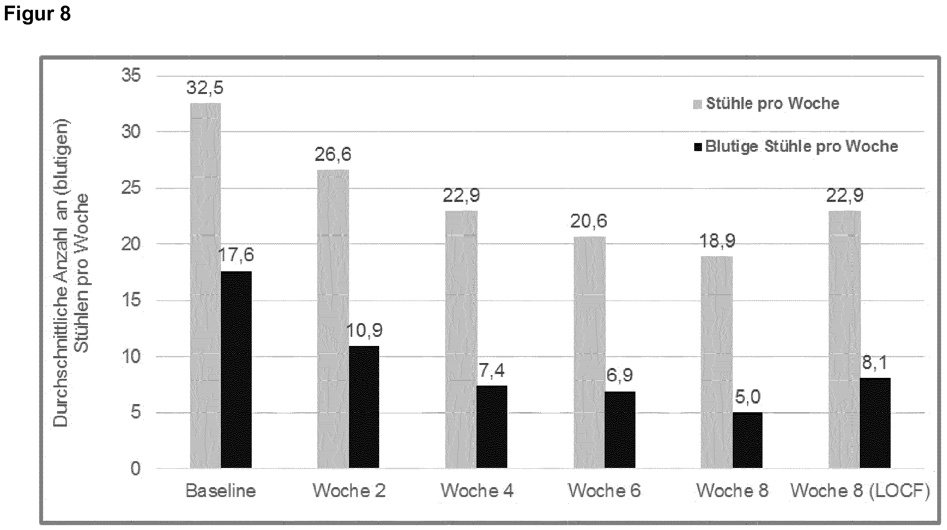

In dieser Studie konnte gezeigt werden, dass die erfindungsgemäße Formulierung tatsächlich über das gesamte Kolon effektiv wirkt (Figur 7). Konsistente Wirksamkeit gemessen über die klinischen Remissionsraten zeigen in den verschiedenen Kolonsegmenten keinen signifikanten Unterschied. Auch die Anzahl Stühle pro Woche und Anzahl der blutigen Stühle pro Woche konnte effektiv gesenkt werden (Figur 8). Die erfindungsgemäße Formulierung konnte in dieser Studie wirksam und sicher angewendet werden. Die Sicherheit der Anwendung wird belegt durch die Messung von morgendlichem Kortisol. Die gemessenen Durchschnittswertewerte blieben im Normbereich von 6.2 - 18 µg/dl.

## Patentansprüche

1. Pellet zur verzögerten Freisetzung des Wirkstoffs insbesondere im distalen Kolon mit einer mehrschichtigen Umhüllung, wobei das Pellet folgende Komponenten aufweist:
a) ein Starter-Pellet, das nur aus einem inerten Material besteht und keinen pharmazeutisch aktiven Wirkstoff im Inneren des Starter-Pellets aufweist,
b) eine Wirkstoffschicht, die direkt auf das Starter-Pellet a) aufgebracht ist und außer dem Wirkstoff filmbildende Hilfsstoffe enthält,
c) eine Quellschicht, die direkt auf die Wirkstoffschicht b) aufgebracht ist und Quellmaterialien enthält, die bei Kontakt mit Darmflüssigkeit aufquellen,
d) eine Retardschicht, die sich bei Kontakt mit der Darmflüssigkeit nicht auflöst, aber für Flüssigkeiten permeabel wird und die direkt auf c) aufgebracht ist,
e) eine äußerste Beschichtung, die sich bei einem pH-Wert <5,5 nicht auflöst, sich aber bei einem pH-Wert, der größer ist als 6,0, gut auflöst, und direkt auf d) aufgebracht ist
wobei der Wirkstoff Budesonid oder ein pharmazeutisch annehmbares Salz davon ist.

2. Pellet gemäß Anspruch 1, **dadurch gekennzeichnet, dass** das Starter-Pellet (a) einen mittleren Durchmesser von 0,2 bis 2,0 mm hat, wobei wenigstens 90 % der Teilchen innerhalb des angegebenen Größenbereichs liegen und wobei die Starter-Pellets eine Kugelform mit gleichmäßiger Oberflächenbeschaffenheit aufweisen.

3. Pellet gemäß Anspruch 2, wobei wenigstens 95 % der Teilchen eine Größenverteilung aufweisen, die innerhalb des Bereichs zwischen 0,2 und 2,0 mm liegen.

4. Pellet gemäß einem der Ansprüche 1-3, **dadurch gekennzeichnet, dass** die Wirkstoffschicht (b) neben dem Wirkstoff Budesonid ein Füllmittel, ein Bindemittel und ein Benetzungsmittel sowie ein Trennmittel enthält.

5. Pellet gemäß einem der Ansprüche 1-4, **dadurch gekennzeichnet, dass** es sich bei dem Wirkstoff um mikronisiertes Budesonid handelt, bei dem die einzelnen Teilchen zu 100 % kleiner als 10 µm sind und zu wenigstens 95 % kleiner als 5 µm sind.

6. Pellet nach einem der Ansprüche 1-5, **dadurch gekennzeichnet, dass** die Quellschicht (c) als Quellmittel homopolymere Polyacrylsäure vom Typ A, ein Bindemittel und ein Trennmittel enthält.

7. Pellet nach einem der Ansprüche 1-6, **dadurch gekennzeichnet, dass** die Retardschicht (d), eine Kombination aus Ammoniummethacrylat-Copolymer (Typ A) und Ammoniummethacrylat-Copolymer (Typ B) ist.

8. Pellet nach einem der vorhergehenden Ansprüche, **dadurch gekennzeichnet, dass** die äußerste Beschichtung (e), die sich bei einem pH-Wert unter 6,0 nicht auflöst, ein Poly(meth)acrylsäure / Poly(meth)acrylat-Copolymerisat mit einem Verhältnis von Poly(meth)acrylsäure zu Polymethylmethacrylat von 1:1 ist.

9. Pellet nach einem der Ansprüche 1 bis 8, **dadurch gekennzeichnet, dass** bei einer in vitro Freisetzung in künstlichem Magensaft mit einem pH-Wert von 1,2 für bis zu 2 Stunden im Wesentlichen keine Freisetzung von Budesonid erfolgt und, dass in künstlichem Darmsaft bei einem pH von 6,5 nach 270 Minuten 10 bis 30 %, nach 330 Minuten 40 bis 70 % und nach 540 Minuten mehr als 80 % des Wirkstoffs Budesonid freigesetzt werden.

10. Pellet nach einem der Ansprüche 1-9, **dadurch gekennzeichnet, dass** in vivo gemäß den Testbedingungen der Pharmakokinetikstudie weniger als 30% des Wirkstoffes innerhalb von vier Stunden freigesetzt werden.

11. Pellet nach einem der Ansprüche 1-10, **dadurch gekennzeichnet, dass** in vivo gemäß den Testbedingungen in der Pharmakokinetikstudie die maximale Plasmakonzentration im Mittel erst nach frühestens 7,0 bis 7,5 Stunden erreicht wird.

12. Im Magen schnell lösliche Kapsel, **dadurch gekennzeichnet, dass** sie eine Vielzahl von Pellets gemäß den Ansprüchen 1-11 enthält.

13. Kapsel nach Anspruch 12, **dadurch gekennzeichnet, dass** es sich um eine im Magen leicht lösliche Gelatinekapsel handelt.

14. Kapsel nach Anspruch 13, **dadurch gekennzeichnet, dass** die darin enthaltenen Pellets 3 mg bis 9 mg Budesonid enthalten.

15. Sachet, **dadurch gekennzeichnet, dass** es so viel Pellets gemäß den Ansprüchen 1 bis 12 beinhaltet, dass in einem Sachet zwischen 3 mg und 9 mg Budesonid enthalten sind.

## Claims

1. A pellet with a multi-layered envelope for retarded release of the active agent, particularly in the distal colon, which pellet has the following components:
a) a starter pellet only consisting of an inert material and having no pharmaceutically effective active agent inside the starter pellet,
b) an active agent layer that is directly applied to the starter pellet a) and in addition to the active agent contains film-forming excipients,
c) a swelling layer that is directly applied to the active agent layer b) and contains swelling materials that swell when contacting intestinal liquor,
d) a retard layer that does not dissolve when contacting the intestinal liquor, but becomes permeable for liquors and that is directly applied to c),
e) an outermost coating that does not dissolve at a pH value < 5.5, but dissolves well at a pH value greater than 6.0 und is directly applied to d);
wherein the active agent is budesonide or a pharmaceutically acceptable salt thereof.

2. The pellet according to claim 1, **characterized in that** the starter pellet (a) has a mean diameter of 0.2 to 2.0 mm, wherein at least 90% of the particles are within the given size range and wherein the starter pellets have a spherical form with uniform surface finish.

3. The pellet according to claim 2, wherein at least 95% of the particles have a size distribution ranging between 0.2 and 2.0 mm.

4. The pellet according to any of claims 1-3, **characterized in that** the active agent layer (b) in addition to the active agent budesonide contains a filler, a binder, and a wetting agent as well as a release agent.

5. The pellet according to any of claims 1-4, **characterized in that** the active agent is micronized budesonide in which 100% of the individual particles are smaller than 10 µm and at least 95% are smaller than 5 µm.

6. The pellet according to any of claims 1-5, **characterized in that** the swelling layer (c) as the swelling agent contains type A homopolymer polyacrylic acid, a binder, and a release agent.

7. The pellet according to any of claims 1-6, **characterized in that** the retard layer (d) is a combination of ammonium methacrylate copolymer (type A) and ammonium methacrylate copolymer (type B).

8. The pellet according to any of the preceding claims, **characterized in that** the outermost coating (e) that does not dissolve at a pH value below 6.0 is a poly(meth)acrylic acid / poly(meth)acrylate copolymer with a ratio of poly(meth)acrylic acid to polymethylmethacrylate of 1:1.

9. The pellet according to any of claims 1 to 8, **characterized in that** substantially no release of budesonide takes place in an in vitro release in artificial gastric juice with a pH value of 1.2 for up to 2 hours and that after 270 minutes 10 to 30%, after 330 minutes 40 to 70% and after 540 minutes more than 80% of the active agent budesonide are released in artificial intestinal juice at a pH of 6.5.

10. The pellet according to any of claims 1-9, **characterized in that** less than 30% of the active agent are released within four hours in vivo according to the test conditions of the pharmacokinetics study.

11. The pellet according to any of claims 1-10, **characterized in that** the maximum plasma concentration on average at the earliest is achieved only after 7.0 to 7.5 hours in vivo according to the test conditions in the pharmacokinetics study.

12. A capsule fast dissolving in the stomach, **characterized in that** it contains a plurality of pellets according to claims 1-11.

13. The capsule according to claim 12, **characterized in that** it is a gelatin capsule that is easily soluble in the stomach.

14. The capsule according to claim 13, **characterized in that** the pellets contained therein contain 3 mg to 9 mg of budesonide.

15. A sachet, **characterized in that** it contains as much pellets according to claims 1 to 12 as that a sachet contains between 3 mg and 9 mg of budesonide.

## Revendications

1. Pastille pour la libération retardée du principe actif, en particulier dans le côlon distal, avec un enrobage multicouche, dans laquelle la pastille présente les composants suivants :
a) une pastille de démarrage, qui est constituée uniquement d'un matériau inerte et ne présente pas de principe pharmaceutiquement actif à l'intérieur de la pastille de démarrage,
b) une couche de principe actif, qui est appliquée directement sur la pastille de démarrage a) et qui contient, outre le principe actif, des adjuvants filmogènes,
c) une couche de gonflement, qui est appliquée directement sur la couche de principe actif b) et qui contient des matériaux de gonflement qui gonflent au contact de liquide intestinal,
d) une couche retardement, qui ne se dissout pas au contact du liquide intestinal, mais qui devient perméable aux liquides et qui est appliquée directement sur c),
e) un revêtement le plus extérieur, qui ne se dissout pas à une valeur pH < 5,5, mais qui se dissout bien à une valeur pH supérieure à 6,0, et qui est appliqué directement sur d)
dans laquelle le principe actif est le budésonide ou un sel pharmaceutiquement acceptable de celui-ci.

2. Pastille selon la revendication 1, **caractérisée en ce que** la pastille de démarrage (a) présente un diamètre moyen de 0,2 à 2,0 mm, dans laquelle au moins 90 % des particules se situent au sein de la plage de tailles indiquée et dans laquelle les pastilles de démarrage présentent une forme sphérique avec une texture de surface uniforme.

3. Pastille selon la revendication 2, dans laquelle au moins 95 % des particules présentent une distribution de tailles qui se situe dans la plage comprise entre 0,2 et 2,0 mm.

4. Pastille selon l'une quelconque des revendications 1-3, **caractérisée en ce que** la couche de principe actif (b) contient, outre le principe actif budésonide, un agent de remplissage, un agent liant et un agent mouillant ainsi qu'un agent de séparation.

5. Pastille selon l'une quelconque des revendications 1-4, **caractérisée en ce que** le principe actif est du budésonide micronisé, dont les particules individuelles sont à 100 % inférieures à 10 µm et sont à au moins 95 % inférieures à 5 µm.

6. Pastille selon l'une quelconque des revendications 1-5, **caractérisée en ce que** la couche de gonflement (c) contient, en tant qu'agent de gonflement, de l'acide polyacrylique homopolymère de type A, un agent liant et un agent de séparation.

7. Pastille selon l'une quelconque des revendications 1-6, **caractérisée en ce que** la couche de retardement (d) est une combinaison de copolymère de méthacrylate d'ammonium (type A) et de copolymère de méthacrylate d'ammonium (type B).

8. Pastille selon l'une quelconque des revendications précédentes, **caractérisée en ce que** le revêtement le plus extérieur (e), qui ne se dissout pas à une valeur pH inférieure à 6,0, est un copolymère acide poly(méth)acrylique/poly(méth)acrylate avec un rapport acide poly(méth)acrylique/polyméthacrylate de méthyle de 1/1.

9. Pastille selon l'une quelconque des revendications 1 à 8, **caractérisée en ce que**, lors d'une libération in vitro dans du suc gastrique artificiel avec une valeur pH de 1,2, il n'y a sensiblement pas de libération de budésonide pendant une durée allant jusqu'à 2 heures, et **en ce que**, dans du suc intestinal artificiel avec un pH de 6,5, 10 à 30 % du principe actif budésonide sont libérés après 270 minutes, 40 à 70 % après 330 minutes et plus de 80 % après 540 minutes.

10. Pastille selon l'une quelconque des revendications 1-9, **caractérisée en ce que**, in vivo, selon les conditions de test de l'étude pharmacocinétique, moins de 30 % du principe actif sont libérés en quatre heures.

11. Pastille selon l'une quelconque des revendications 1-10, **caractérisée en ce que**, in vivo, selon les conditions de test de l'étude pharmacocinétique, la concentration plasmatique maximale n'est atteinte en moyenne qu'après au plus tôt 7,0 à 7,5 heures.

12. Capsule rapidement soluble dans l'estomac, **caractérisée en ce qu'**elle contient une pluralité de pastilles selon les revendications 1-11.

13. Capsule selon la revendication 12, **caractérisée en ce qu'**il s'agit d'une capsule de gélatine facilement soluble dans l'estomac.

14. Capsule selon la revendication 13, **caractérisée en ce que** les pastilles qu'elle contient contiennent de 3 mg à 9 mg de budésonide.

15. Sachet, **caractérisé en ce qu'**il contient suffisamment de pastilles selon les revendications 1 à 12 pour qu'entre 3 mg et 9 mg de budésonide soient contenus dans un sachet.
